# EUROPEAN PATENT APPLICATION

(11) **EP 2 336 102 A1**
(43) Date of publication of application: **22.06.2011**
(21) Application number: 11000266.4
(22) Date of filing: 03.11.2006
(51) Int. Cl.: C07C 17/20, C07C 17/25, C07C 17/10, C07C 17/02, C07C 21/18, C07C 19/08, C07C 19/10

(54) **Method for producing fluorinated organic compounds**

(30) Priority: 03.11.2005 US 733383 P; 03.11.2005 US 733444 P
(62) Divisional of application: 06844259.9
(71) Applicant: Honeywell International Inc., Morristown, NJ 07962-2245 (US)
(72) Inventor: Merkel, Daniel, C, Morristown NJ 07960 (US); Tung, Hsueh, Suung, Morristown NJ 07960 (US); Nair, Haridasan, K., Morristown NJ 07960 (US); Van der Puy, Michael, Morristown NJ 07960 (US); Ma, Jing, Ji, Morristown NJ 07960 (US); Dubey, Rajesh, Morristown NJ 07960 (US); Light, Barbara, Morristown NJ 07960 (US); Fleming, Kim, Morristown NJ 07960 (US); Bortz, Cheryl, Morristown NJ 07960 (US); Udy, Richard, Morristown NJ 07960 (US); Mukhopadhyay, Sudip, Morristown NJ 07960 (US)
(74) Representative: Hucker, Charlotte Jane

(57) **Abstract**

A method for preparing fluorinated organic compounds comprising contacting hydrogen fluoride with at least compound of Formula (I): CX₃CXYCH₃, where each X is independently Cl, I or Br, and each Y is independently H or F, said contacting step being carried out under conditions effective to produce a compound of Formula (II): CF₃CZ CH₂, where Z is Cl, I, Br or F.

## Description

### BACKGROUND

### (1) Field of The Invention:

This invention relates to novel methods for preparing fluorinated organic compounds, and more particularly to methods of producing fluorinated olefins.

### (2) Description of Related Art:

Hydrofluorocarbons (HFC's), in particular hydrofluoroalkenes such as tetrafluoropropenes (including 2,3,3,3-tetrafluoro-1-propene (HFO-1234yf)) have been disclosed to be effective refrigerants, fire extinguishants, heat transfer media, propellants, foaming agents, blowing agents, gaseous dielectrics, sterilant carriers, polymerization media; particulate removal fluids, carrier fluids, buffing abrasive agents, displacement drying agents and power cycle working fluids. Unlike chlorofluorocarbons (CFCs) and hydrochlorofluorocarbons (HCFCs), both of which potentially damage the Earth's ozone layer, HFCs do not contain chlorine and thus pose no threat to the ozone layer.

Several methods of preparing hydrofluoroalkanes are known. For example, U.S. Pat. No. 4,900,874 (Ihara et al) describes a method of making fluorine containing olefins by contacting hydrogen gas with fluorinated alcohols. U.S. Pat. No. 2,931,840 (Marquis) describes a method of making fluorine containing olefins by pyrolysis of methyl chloride and tetrafluoroethylene or chlorodifluoromethane. The preparation of HFO-1234yf from trifluoroacetylacetone and sulfur tetrafluoride has been described. *See* Banks, et al., Journal of Fluorine Chemistry, Vol. 82, Iss. 2, p. 171-174 (1997). Also, U.S. Pat. No. 5,162,594 (Krespan) discloses a process wherein tetrafluoroethylene is reacted with another fluorinated ethylene in the liquid phase to produce a polyfluoroolefin product.

Notwithstanding prior teachings, applicants have come to appreciate a continuing need for methods of efficiently preparing certain hydrofluorocarbons, particularly tetrafluorpropenes such as HFO-1234yf.

### SUMMARY OF THE INVENTION

Applicants have developed methods for producing fluorinated organic compounds, including hydrofluoropropenes, which preferably comprises contacting at least one compound of the formula (XₐY₁₋ₐ)F with at least one compound of formula (I):

CX₃CXYCH₃ (I)

where each X is independently Cl, I or Br, each Y is independently H or F and a is 0 or 1.

The preferred contacting step of the present invention is carried out under conditions effective to produce a compound of formula (II)

CF₃CZ=CH₂ (II)

where Z is Cl, I, Br, or F.
In certain preferred embodiments the contacting step produces a reaction product comprising tetrafluoropropene, and in particular 2,3,3,3-tetrafluoro-1-propene (HFO-1234yf).

The contacting step preferably comprises reacting a compound of formula (I) with at least one compound of the formula (XₐY₁₋ₐ)F, such as HF, in the gas phase in the presence of a catalyst. In preferred embodiments, the conversion of the compound of formula I in the reaction is from about 70% to about 100 %. In certain embodiments, including those which produce conversion levels as indicated herein, the selectivity of the reaction to tetrafluoropropene, and to HFO-1234yfin particular, is from about 5 to about 40%. In certain preferred embodiments the reaction product also contains a trifluor-monochloropropene, particularly 2-chloro, 3,3,3,trifluoro-1-propoene (HFO-1233xf) with a selectivity of from about 50% to about 90%.

### DETAILED DESCRIPTION OF PREFERRED EMBODIMENTS

One beneficial aspect of the present invention is that it enables the production of desirable fluroolefins, preferably C3 fluoroolefins, from relatively low cost and readily obtainable starting materials. More specifically, certain preferred embodiments of the present invention involve producing the desired C3 fluoroolefin from C3 olefin's, such as propylene. Propylene is in many embodiments an advantageous starting material because it is relatively inexpensive, is relatively easy to handle, and is generally readily available in commercial quantities.

Thus, in certain embodiments the present methods include the step of reacting C3 olefin, such as propylene, with a halogenating agent (such as CIF or HF) under conditions effective to produce a compound of formula (III)

CH₃₋ₙXₙCXYCH₃ (III)

where n is 0, 1 or 2. This reaction is sometimes referred to herein for convenience but not necessarily by way of limitation, as a halogen addition reaction.

Preferably, the compound of formula (III) is then reacted, preferably in a photo-chlorination reaction, to produce a reaction product containing at least one compound of formula (I) as described above. Preferably the formula (I) compound is then exposed to reaction conditions, which are sometimes referred to herein for convenience, but not necessarily by way of limitation, as fluorination to produce a reaction product containing one or more of the desired fluorolefins. Preferred aspects of each of these preferred steps is described below, with the titles used as headings for these steps being used for convenience but not necessarily by way of limitation.

### A. REACTION OF C3 OLEFIN WITH HALOGENATING AGENT

In preferred embodiments, a compound of formula (IV)

CHₙX₃₋ₙCHC=CH₂ (IV)

is contacted under reaction conditions with a compound of formula XₘY₂₋ₘ wherein X, Y are as defined above, provided that the compound is not H₂, and m is 0, 1 , or 2, to produce a compound of formual (III), namely, CHₙX₃₋ₙCXYCH₃, where n is 0, 1 or 2. In preferred embodiments, the compound of formula (IV) is propylene (n is 3 and X is H) and the compound of formula XₘY₂₋ₘ is one or more of CIF, Cl₂, F₂ and HF, preferably one or more of CIF, Cl₂, and HF. The compound XₘY₂₋ₘ, and compounds which perform a similar function in the present reactions, are referred to herein as halogenation agents.

In certain preferred embodiments, the contacting step comprises contacting, (preferably by introducing into a reactor) the compounds in an halogenation agent:formula (IV) mole ratio of from about 1:1 to about 4: 1, and even more preferably of from about 2:1 to about 4:1. In preferred embodiments in which the compound of XₘY₂₋ₘ comprises HF and the formula (IV) compound comprises propylene, the HF:propylene mole ratio of the feeds to the reactor are from about 1:1 to about 10:1 and even more preferably from about 2:1 to about 4:1. In preferred embodiments in which the compound of XₘY₂₋ₘ comprises CIF (X is Cl, Y is F, and m is 1) and the formula (IV) compound comprises propylene, the CIF:propylene mole ratio of the feeds to the reactor are from about 10 to about 0.1 and even more preferably from about 1 to about 0.2. In preferred embodiments in which the compound of XₘY₂₋ₘ comprises Cl₂ (m is 2 and X is Cl) and the formula (IV) compound comprises propylene, the Cl₂:propylene mole ratio of the feeds to the reactor are from about 5 to about 0.1 and even more preferably from about 1 to about 0.2.

This reaction step can be carried out in the liquid phase and/or in the gas phase, and it is contemplated that the reaction can be carried out batch wise, continuous, or a combination of these.

### 1. LIQUID PHASE HALOGEN ADDITION REACTIONS

Although not necessarily preferred, certain embodiments of the present invention (particularly when the XₘY₂₋ₘ compound is CIF, HF or Cl₂, or combinations of two or more of these) involve relatively low temperature reactions in which at least the organic reactant(s) are charged to the reactor as liquids, with the reactor preferably maintained at a temperature of from about -90°C to about -50°C, and at least a portion of the reaction is carried in the liquid phase (the normal boiling point HₙX₃₋ₙof the preferred reactant propylene is - 47.8 °C). However, it is contemplated that at least some portion of the reaction product may be produced and/or removed from the reaction mixture in such embodiments as a gaseous material.

For those preferred embodiments which utilize CIF as a reactant, it is sometimes preferred to provide CIF, preferably in situ, by the liquid-phase reaction of HF and Cl₂ in the presence of catalyst, preferably a transition metal catalyst, and even more preferably a transition metal halide catalysts such as FeC13, TaCl5, TiCl4, SbCl5, SbCl3, and CrCl3, SbF3, SbF5, AlF3, and CrF3, and combinations of two or more of these. In certain preferred embodiments, therefore, the present halogenation step comprises contacting CH3CH=CH2, HF and Cl2 in the presence of a metal catalyst, preferably a metal chloride salt, preferably with addition of kinetic energy to provide a substantially uniform reaction mixture (such as stirring), under conditions effective to form a reaction product comprising the desired compound of formula (III).

In certain preferred embodiments, the reaction is carried out at a temperature of from about -90°C to about -50°C, more preferably from about -80°C to about - 70°C, under conditions effective to achieve a percentage conversion of at least about 70%, more preferably at least about 90%, and even more preferably at least about 100% of the compound of formula (IV). Preferably, the reaction conditions are effective to achieve a percentage selectivity to compounds of formula (III), and preferably 2-fuoropropane, of at least about 50%, more preferably at least about 75%, and even more preferably at least about 95%. In certain preferred embodiments a selectivity of about 98% or greater is achieved.

As used herein, the term "percentage conversion" with respect to a reactant refers to the moles reacted in the reaction process divided by the moles of reactant in the feed to the process multiplied by 100.

As used herein, the term "percentage selectivity" with respect to an organic reaction product refers to the ratio of the moles of that reaction product to the total of the remaining organic reaction products multiplied by 100.

In certain preferred embodiments the reaction time for the preferred liquid phase halogenation reaction is from about 1 to about 3 hours. The reaction product in preferred embodiments includes one or more of CH₃CHFCH₂Cl, CH₃CHClCH₂CI, CH₃CHCICH₂Cl, and/or CH₃CHFCH₃. In preferred embodiments, the reaction product comprises from about 40 wt. % to about 75 wt.% CH₃CHFCH₂Cl, from about 5 to about 35 wt. % CH₃CHClCH₂CI, from about 5 to about 15 wt% CH₃CHCICH₂Cl, and less than about 5% CH₃CHFCH₃.

It will be appreciated that many alternatives for the provision of CIF in accordance with this preferred step of the present invention are available and within the scope hereof. By way of example, the reactant CIF may be provided in certain embodiments simply by purchasing the needed quantity of the material in the appropriate form. In other preferred embodiments, it is desirable to provide the CIF by conducting a liquid-phase reaction of HF and Cl₂, preferably in the presence of transition metal halide such as SbF₅. Such reactions, especially single stage reactions, can be achieved using any equipment and conditions known and available in the art for such types of reactions, preferably at a temperature of from about -50 °C to -90°C, and even more preferably the reaction temperature is maintained at a temperature of from about -65°C to about -85°C.

In certain embodiments, a two stage reaction may be used, with the first stage being a gas phase reaction and the second stage being a liquid phase reaction. In such embodiments the CIF produced in this first reaction stage, and/or from other sources, is preferably then charged to a second reaction vessel or to a second region of the same vessel, preferably a second autoclave, where it is contacted with a compound of formula (IV), such as propylene, at a temperature as specified above, preferably at about -75°C, and preferably under conditions capable of providing selectivity to a compound of formula (III0, preferably CH₃CHFCH₂CI, of at least about 60% and up to about 75% or greater.

### 2. GAS/LIQUID PHASE HALOGEN ADDITION REACTIONS

Although not necessarily preferred, the formation of a compound of formula III may also be carried out at least partially in a gas phase reaction, preferably in the presence of a catalyst. For example, high conversion and selectivity can be achieved in some embodiments by first reacting HF and Cl₂ in a liquid phase, and preferably in a continuous liquid phase reaction, by charging the reactor with a catalyst, preferably a transition metal halide such as SbF₅ and conducting the reaction at a temperature of from about -50 °C to 50°C to produce ClF. In such embodiments it is preferred to introduce the ClF produced in the gas phase, together with a compound of formula (IV), into a liquid phase reactor, generally in accordance with the conditions described above, to produce a compound of formula (III), preferably at a selectivity to CH₃CHFCH₂CI of at least about 60%, and even more preferably at least about 70%.

### 3. PREFERRED GAS PHASE HALOGENATION REACTIONS

For certain preferred embodiments, particularly those preferred embodiments which utilize HF as a reactant, it is preferred to provide a compound of formula (III) by a gas-phase reaction of HF and a compound of formula (IV) in the presence of catalyst, preferably a transition metal catalyst, and even more preferably a transition metal halide catalysts such as FeCl₃, TaCl₅, TiCl₄, SbCl₅, SbCl₃, and CrCl₃, SbF₃, SbF₅, AlF₃, and CrF₃, and combinations of two or more of these. In certain preferred embodiments, therefore, the preferred halogenation step comprises contacting CH₃CH=CH₂, preferably an iron based catalyst, and even more preferable an iron chloride catalyst, and maintaining the reactor temperature at from about 20°C to about 100°C, more preferably from about 25°C to about 35°C, with a preferred reactor pressure of from about 15 to about 200 psia, and even more preferably from about 20 to about 100 psia. In such preferred embodiments HF is preferably charged to the reactor, preferably as a liquid (for example by maintiaing the vessel containing the HP under pressure, such as 45 psig of N₂ head space pressure) and the compound of formula (IV) is charged to the reactor, preferably as a gas. In such embodiments the conversion of the formula (IV) compound, particularly propylene, is preferably at least about 40%, more preferably at least about 80%, and selectivity to compounds of formula (III), particularly 2-fluoropropane, is preferably at least about 40%, more preferably at least about 60%, more preferably at least about 80%, and in certain embodiments at least about 90%. In certain preferred embodiments selectivity of about 95% is preferred. Examples of other catalysts that may be used in this aspect of the invention include carbon, carbon nanotubes, SbCl₅IC, SbF₅/C, FeCI₃, CrF₃, Cr-oxyfluoride, and Cr₂03.

### B. CHLORINATION OF THE COMPOUND OF FORMULA III

According to preferred aspects of this invention, a compound of formula (III), preferably but not necessarily produced in accordance with the procedures described above, is chlorinated to produce a compound of formula (I), namely, CX₃CXYCH₃ where each X is independently Cl, I or Br, and each Y is independently H or F. In its broad aspects, the preferred chlorination step is amenable to a large number of specific processing condition and steps in accordance with the teachings contained herein, and all such variations are within the broad scope of the present invention. It is particularly preferred, however, that the chlorination step comprise photochlorination. Thus, the preferred reaction step comprises exposing the reactants, preferably in a liquid phase, to ultraviolet radiation, preferably in the range of from about 200 to about 400nm. The chlorination agent preferably comprises chlorine gas, either neat or preferably with a diluent such as nitrogen. A chorination catalyst, such as V₂O₅, Zeolites, and Au/Ti0₂ catalyst, may be used in certain embodiments. The reaction is preferably carried out a temperature of from about -20°C to about 200°C, and even more preferably from about 25°C to about 120°C for a time of from about 5 seconds to about 5 hours, more preferably from about 15 seconds to about 30 min. The reaction product, which comprises a compound of formula (I), may then optionally be subject to one or more separation steps, such as distillation, to remove unwanted byproducts and produce a stream relatively concentrated in compounds of the formula (I).

### C. FORMATION OF THE COMPOUND OF FORMULA II

The methods of the present invention preferably comprise reacting a compound of formula (I) with a fluorinating agent to produce a fluorolefin, prefereably a C3 fluorolefin, more preferably a compound of formula (II), and even more preferably tetrafluoropropene. This preferred reaction step may be described, by way of illustration but not necessarily by way of limitation, by the following reaction equation in connection with embodiments in which the compound of formula (I) is tetrafluormonochloropropane and the fluorinating agent is hydrogen fluoride:

CCI₃CFClCH₃ + 3HF → CF₃CF=CH₂ + HCl

This aspect of the present invention is sometimes referred to herein as a fluorination reaction. In many aspect of such preferred embodiments, CF₃CCl=CH₂ (HFO-1233xf) is also produced in the reaction.

It is contemplated that this reaction step may be preformed using a wide variety of process parameters and process conditions in view of the overall teachings contained herein. For example, it is contemplated that certain embodiments may comprise a liquid phase, non-catalytic reaction. However, it is generally preferred that this reaction step comprise a gas phase catalyzed reaction, preferably a metal catalyst, and even more preferably one or more transition metal-based catalysts (including in certain preferred embodiments transition metal halide catalysts), such as FeCl₃, chromiumoxyfluoride, Ni (including Ni mesh), NiCl₂, CrF₃. Other catalysts include carbon-based catalysts, antimony-based catalysts (such as Sb/Cl₅), aluminum-based catalyst (such as AlF₃ and AlO₃). Many other catalysts may be used, including palladium-based catalyst, platinum-based catalysts, Rhodium-based catalysts and ruthenium-based catalysts. Of course, two or more any of these catalysts, or other catalysts not named here, may be used in combination.

In general it is preferred that the catalysts are pretreated by fluorination prior to use. In preferred embodiments, fluorination of the catalysts comprises exposing the catalyst to a stream of HF at about reaction temperature and pressure.

In certain preferred embodiments, the present step of fluorinating a compound of formula I to produce a compound of formula II comprises contacting a the formula I compound with a fluorinating agent, preferably under conditions effective to provide a formula I conversion of at least about 10%, more preferably at least about 55%, and even more preferably at least about 70%. In certain preferred embodiments the conversion is at least about 90%, and more preferably about 100%. Furthermore, in certain preferred embodiments, the present step of fluorinating a compound of formula I to produce a compound of formula II is conducted under conditions effective to provide a formula II selectivity of at least about 5%, more preferably at least about 20%, more preferably at least about 50%, and even more preferably at least about 90%. In embodiments in which the compound of formula I comprises CCl₃CFClCH₃ (HCFC-241bb), the selectivity to HFO-1234yfis at least about 5%, more preferably at least about 10%, more preferably at least about 50% or higher, and the selectivity to HFO-1233xfis at least about 5%, more preferably from about 30% to about 70%, and even more preferably from about 40% to about 60%.

### EXAMPLES

Additional features of the present invention are provided in the following examples, which should not be construed as limiting the claims in any way. Examples 1 - 24:
These examples illustrate gas phase fluorination-dehydrohalogenation of CCI₃CFClCH₃ (241bb) to CF₃CCl=CH₂ (12334) and CF₃CF=CH₂(1234yf).

A 22-inch (1/2-inch diameter) Monel tube reactor is charged with 120 cc of catalyst, as specified in Table I below. All catalysts are fluorinated for 6 h with 80 glh of HF at reaction temperature under 20 psig pressure. The reactor is mounted inside a heater with three zones (top, middle and bottom). The reactor temperature is monitored using a 5-point thermocouples kept at the middle inside of the reactor. The inlet of the reactor is connected to a pre-heater, which is kept at about 300°C by electrical heating. Liquid HF is fed from a cylinder into the pre-heater through a needle valve, liquid mass-flow meter, and a control valve at a constant flow of 1-1000 g/h. The HF cylinder is kept at a constant pressure of about 40 psig by applying anhydrous N₂ gas pressure into the cylinder head space. 10-120 g/h of organic compound of formula I (HCFC-241bb) is fed from a cylinder kept at about 145°C through a regulator, needle valve, and a gas mass-flow-meter. The compound of formula I is also fed time to time as liquid at 120°C from a cylinder into the pre-heater through a needle valve, liquid mass-flow meter, and a control valve at a constant flow of 50-150 g/h. The organic line from the cylinder to the pre-heater is kept at 265°C by wrapping with constant temperature heat trace and electrical heating. All feed cylinders are mounted on scales to monitor their weight by difference. The reactions are run at a constant reactor pressure of about 0-100 psig by controlling the flow of reactor exit gases by another control valve. The gases exiting the reactor are analyzed by on-line GC and GC/MS connected through a hotbox valve arrangement to prevent condensation. The product was collected by flowing the reactor exit gases through a 20-60 wt% aq. KOH scrubber solution and then trapping the exit gases from the scrubber into a cylinder kept in dry ice or liquid N₂. The products were then isolated by distillation. The results are shown in Table I below.

**Table 1: CCl₃CFCICH₃ + 3HF → CF₃CF=CH₂ + HCl**

| Example# / Catalyst | T, C | % Conversion of 241bb | % Selectivity to 1234yf | % Selectivity to 1233xf | % Selectivity 1234yf/1233xf |
|---|---|---|---|---|---|
| Example 1/ Chromium Oxoxyfluoride | 250 | 56 | 1 | 12 | 0.08 |
| Example 2/ (Plant Catalyst) | 320 | 92 | 7 | 85 | 0.08 |
| Example 3/ (Plant Catalyst) | 350 | 96 | 8 | 69 | 0.11 |
| Example 4/ (Plant Catalyst) | 375 | 100 | 12 | 63 | 0.19 |
| Example 5/ 4-6wt%FeCI3/C | 50 | Plugged | | | |
| Example 6/ (NORIT-RFC-3) | 100 | Plugged | | | |
| Example 7 | 120 | 100 | 14 | 57 | 0.25 |
| Example 8 | 150 | 100 | 14 | 61 | 0.23 |
| Example 9 | 200 | 100 | 11 | 54 | 0.2 |
| Example 10 | 220 | 100 | 9 | 43 | 0.2 |
| Example 11/ Carbon | 300 | 56 | 11 | 26 | 0.42 |
| Example 12/ Carbon | 400 | 71 | 9 | 17 | 0.53 |
| Example 13/ Carbon | 500 | 89 | 5 | 14 | 0.36 |
| Example 14/ Ni-mesh | 250 | 42 | 7 | 29 | 0.24 |
| Example 15/ Ni-mesh | 350 | 84 | 10 | 62 | 0.16 |
| Example 16/ Ni-mesh | 425 | 100 | 14 | 53 | 0.26 |
| Example 17/ 25 wt% SbCls/C | 110@50 psig | 100 | 18 | 59 | 0.3 |
| Example 18/ 25 wt% SbCls/C | 120@50 psig | 100 | 21 | 49 | 0.42 |
| Example 19/ 14wt% NiC12/Al2O3 | 250 | | | | |
| Example 20/ 1.4wt% NiC12/Al2O3 | 320 | 92 | 14 | 62 | 0.22 |
| Example 21/ 1.4% NiC12/Al2O3 | 350 | | | | |
| Example 22/ 1.4wt%Ni/Al2O3 | 350 | 100 | 6 | 52 | 0.12 |
| Example 23/ AlF3 | 150 | 82 | 0 | 8 | 0 |
| Example 24/ CrF3 | 200 | 89 | 2 | 11 | 0.18 |

In addition to the products identified in the table, other compounds produced in the reaction are 1,1,1-trifluoroethane (143a), 3-chloro-2,2,3,3-tetrafluoropropane(244cc), 3,3-dichloro-2,2,3-trifluoropropane(243cc), I-propene-1,1-dichloro-2-fluoro, 2,3,3-trichloro-2-fluoropropane(251bb), 1-chloro-3,3,3-trifluoro-1-propene (1233zd), 3,3,3-trifluoro-1-propene (1243zf),1,1-dichloro-2-fluoro-1-propene,2,3,3-trichloro-2-fluoropropane, dichlorodifluoropropene, and carbon black.

### Example 25

This example illustrates addition of F₂ to CH₃CH=CH₂ in a liquid phase reaction, which is illustrated by the following reaction scheme:

CH₃CHCl₂ + F₂ → CH₃CHFCH₂F

About 1-100 wt% F₂ in nitrogen is bubbled through 125 g of liquid propylene in a stirred Hastrelloy C reactor at about -50°C to -75°C for about 1 hour in the presence of HF as the solvent. A 1 gallon Parr reactor is first charged with a relatively inert solvent, HF, to help with heat transfer and dilution of the organic. Then 125 grams of propylene are added batch wise to the reactor. The reaction mixture is continuously mixed and cooled to the desired temperature. Then the F₂ feed (1 wt%), diluted with N2 (99 wt%), is introduced continuously directly into the reaction mixture through a dip tube until about 90% of the stoiciometric amount needed to convert all the propylene that is added. The reactor temperature and pressure are controlled automatically at the desired set points of between -50 to -75°C and a constant pressure of 40 psig. The temperatures are chosen to minimize the amount of propylene that would exit the reactor with the N₂ diluent. The gases exiting the reactor are passed through a caustic scrubber carboy and an activated alumina column to remove acidity, then a dri-rite column to remove moisture, and finally the organic is collected in a DIT. When the desired amount of F₂ is added the reaction liquid is sampled. The sample is absorbed in H₂O and the organic is recovered by phase separation. The organic is then analyzed by GC and GC/MS. The remaining material in the reactor is boiled off through the scrubbing system and the organic is collected in the DIT and analyzed by GC and GC/MS. The analyses are used to determine that the reaction has an overall selectivity to CH₃CHFCH₂F of about 36-45%.

### Example 26

This example illustrates addition of F₂ to CH₃CH=CH₂ in a gas phase reaction, which is illustrated by the following reaction scheme:

CH₃CH Cl₂ + F₂→ CH₃CHFCH₂F

The same apparatus as described in Example 25 is used, except that gaseous propylene and 1% F2 (99% dilution w/ N2) are fed into the Parr reactor via a common dip tube. Propylene is fed at a 50% stoichiometric excess. The reactor is kept at 70°C and at atmospheric pressure. The reactor effluent is passed through a DIT, which collected most of the organic. Only a couple of grams of vapor are left in the Parr reactor at the end of the experiment. GC analysis of the material indicated about 10% conversion of the propylene. The selectivity to CH₃CHFCH₂F is about 32% based on GC area%.

### Example 27

This example illustrates addition of F2 to CH3CH=CH2 in a gas phase reaction under the same conditions as Example 26, except the reaction is performed at higher temperature of about 0 to -20°C. A yield of about 10% CF₃CHFCH₂F is obtained, which is then transformed to HFO-1234yf with 100% selectivity in the next step by reacting with 20%KOH solution in the presence of 18-crown ether as the phase-transfer catalyst at about 55°C using a 300 ml autoclave.

### Example 28

This example illustrates addition of fluorine to CH₃CH=CH₂ in a gas phase reaction, which is illustrated by the following reaction scheme:

CH₃CH Cl₂ + HF → CH₃CHFCH₃

In a gas-phase reaction a Monel tube reactor was charged with 1909 cc of 4-6-wt% FeCl₃/C (NORIT-RFC 3, commercial catalyst from NORIT NEDERLAND B.V.). The reactor was mounted in a constant temperature sand-bath with 40-50 ml/min of air flowing through the sand-bath. The sand-bath set point was set at 28°C during the reaction. The inlet of the reactor was connected to a pre-heater which was kept at 106°C by supplying 30 psig steam through the jacket. The liquid-HF was fed from a cylinder into the pre-heater through a positive displacement pump and kept at a constant flow of 15.88 mol/h (318 g/h) by controlling the flow with a research control valve. The HF cylinder was kept at a constant pressure of 30-40 psig by applying anhydrous N₂ gas pressure into the cylinder head space. A 5.4 mol/h (227 g/h) of propylene is fed as a gas from a cylinder through a regulator, needle valve, and flow controller directly into the reactor inlet at a point just after the pre-heater. HF and Propylene cylinders were mounted on two different scales to monitor their weight by difference. The reactions were run at a constant reactor pressure of 25 psig by controlling the flow of reactor exit gases by another research control valve. The mole ratio of HF to Propylene was kept at 2.94 with a contact time of 33 sec. The exit gases coming out of the reactor were analyzed by an on-line GC and GCMS connected through a hotbox valve arrangements to prevent condensation. The conversion of Propylene was almost 100% and the selectivity to 2-Fluoropropane was 98%. The reaction was performed continuously over 4 days period and the catalyst did not loose any activity. The product was collected by flowing the reactor exit gases through a 20-60 wt% aq. KOH scrubber solution and then trapping them in a cylinder kept inside dry ice or liquid N₂.

### Examples 29 - 42

This example illustrates the chlorination of 2-fluoropropane (CH₃CHFCH3) to CC 13 CFC 1 CH3 (24 1 bb) in a gas phase reaction, which is illustrated by the following reaction scheme:

CH₃CHFCH₃ + 4Cl_{2 → (light)} CCl₃CFClCH₃ + 4HCl

A 500 ml Pyrex reactor was placed inside a Rayonet photo chlorinator at room temperature (25°C). The reactor used in this study was a RAYONETModel-RPR-100 with standard operating temperature of 35°C and standard power consumption around 400 watts. The inside of the photo chlorinator was equipped with 16 UV-lamps; the number of lamps could be varied easily. The 2-Fluoropropane was fed into the reactor from a cylinder kept at a constant water bath temperature of 35°C through a regulator and mass-flowcontroller. Chlorine gas was fed from a cylinder through a regulator, needle valve, and a massflow-controller into the reactor. N₂ was used as the diluents, though the reaction proceeds well without it from a cylinder, which was fed from a cylinder through regulator and mass-flowcontroller. A fan at the bottom of the photo chlorinator was kept on with a constant flow of N₂ to helped keeping the reactor at a constant temperature. This was because of fast dissipation of heat of reaction to avoid run-away reaction. The products that were high boiling liquids stayed inside the reactor collection section, collected and analyzed after the stipulated reaction time. The exit gases out of the reactor was analyzed by on-line GC which contains mainly unreacted 2-fluoropropane, N2 and mono-chlorinated products. The liquid products thus obtained was then washed with water to remove HCl and soluble Chlorine and then purified by distillation under vacuum. The experimental mp of 241bb is 98-101°C and boiling is 139°C. The purity was around 95-100%. The results are shown in Table 2 below. -

**Table 2 - Photo chlorination of 2-Fluoropopane (R281ea) to CCl₃CFClCH₃ (241bb)**

| Ex # | FP, Sccm | Cl₂, sccm | N₂, sccm | Contact time, sec | Mole ratio of Cl₂ to FP | T, h | Lamp | %Conversion | %Sel. to CCl₃CF CICH₃ | %Overall sel. |
|---|---|---|---|---|---|---|---|---|---|---|
| 29 | 20 | 96 | 0 | 206 | 4.8 | 2 | 8 | 48 | 18 | 49 |
| 30 | 20 | 80 | 0 | 239 | 4 | 2 | 8 | 52 | 29 | 62 |
| 31 | 20 | 80 | 50 | 159 | 4 | 1 | 8 | 54 | 38 | 71 |
| 32 | 20 | 80 | 100 | 120 | 4 | 1 | 8 | 46 | 19 | 63 |
| 33 | 20 | 120 | 50 | 126 | 6 | 1 | 8 | 75 | 35 | 69 |
| 34 | 20 | 150 | 50 | 108 | 7.5 | 1 | 8 | 34 | 19 | 81 |
| 35 | 20 | 20 | 76 | 206 | 1 | 1 | 8 | 27 | 26 | 85 |
| 36 | 20 | 40 | 56 | 206 | 2 | 1 | 8 | 35 | 20 | 81 |
| 37 | 20 | 60 | 36 | 206 | 3 | 1 | 8 | 35 | 28 | 82 |
| 38 | 20 | 80 | 16 | 206 | 4 | 1 | 8 | 42 | 32 | 74 |
| 39 | 20 | 96 | 0 | 206 | 4.8 | 1 | 8 | 50 | 42 | 73 |
| 40 | 20 | 96 | 0 | 206 | 4.8 | 0.25 | 8 | 45 | 35 | 70 |
| 41 | 20 | 96 | 0 | 206 | 4.8 | 0.5 | 8 | 40 | 37 | 76 |
| 42 | 20 | 96 | 0 | 206 | 4.8 | 0.75 | 8 | 54 | 41 | 71 |

| | | | | | | | | | | |
|---|---|---|---|---|---|---|---|---|---|---|
| *FP is 2-Fluoropropane; sccm is standard cubic centimeter per minute; total volume of the reactor in contact with the light is 450 ml (Pyrex); Conversion is the ratio of moles of FP converted to products to total moles of FP fed; Selectivity to CCl₃CFC1 CH₃ is the ratio of moles of FP converted to CCl₃CFClCH₃ to total moles of FP reacted; Overall selectivity is the ratio of moles of FP converted to CH₃CFCICH₃, CICH₂CFCICH3, Cl₂CHCFCICH₃, and CCl₃CFClCH₃ to total moles of FP reacted. There are total 16 lamps. The reactor is a BAYONET-Model-RPR-100, standard operating temperature 35°C and standard power consumption 400 watts,* | | | | | | | | | | |

### Examples 43 - 72

This example illustrates the photochlorination of 2-fluoropropane (CH₃CHFCH₃) to CCl₃CFClCH₃ (241bb) in a gas phase reaction as described in Examples 29-42, except 4 parameters are varied, including flow rates of Cl₂ and 2-Fluoropropane, time of irradiation, and number of lamps at room temperature. The results are reported in Table 3 below:

**TABLE 3 - Photo chlorination of 2•Fluoropopane to CCl₃CFClCH₃**

| Ex # | Cl₂, Scc m | 2-FP, sccm | Time, | No of lamps | N₂, sccm | Contact time, sec | Mole ratio of Cl₂ to 2-FP | % 2-FP Conversion | %Sel. to CCl₃CF ClCH₃ | %Overall sel to mono-, di-, tri-and tetra-chloro compound S |
|---|---|---|---|---|---|---|---|---|---|---|
| 43 | 80 | 40 | 20 | 4 | 25 | 165 | 2 | 65 | 57 | 68 |
| 44 | 80 | 20 | 20 | 4 | 25 | 191 | 4 | 70 | 44 | 74 |
| 45 | 50 | 30 | 30 | 6 | 25 | 228 | 1.7 | 31 | 54 | 67 |
| 46 | 50 | 30 | 30 | 6 | 25 | 228 | 1.7 | 31 | 54 | 67 |
| 47 | 20 | 20 | 20 | 4 | 25 | 367 | 1 | 54 | 24 | 90 |
| 48 | 20 | 40 | 20 | 8 | 25 | 281 | 0.5 | 34 | 14 | 87 |
| 49 | 20 | 40 | 40 | 4 | 25 | 281 | 0.5 | 33 | 18 | 93 |
| 50 | 80 | 40 | 40 | 8 | 25 | 281 | 0.5 | 54 | 48 | 57 |
| 51 | 80 | 40 | 20 | 8 | 25 | 165 | 2 | 100 | 55 | 61 |
| 52 | 20 | 40 | 20 | 4 | 25 | 281 | 0.5 | 22 | 1.8 | 78 |
| 53 | 80 | 20 | 40 | 4 | 25 | 191 | 4 | 72 | 50 | 57 |
| 54 | 80 | 20 | 20 | 8 | 25 | 191 | 4 | 96 | 28 | 43 |
| 55 | 80 | 40 | 40 | 4 | 25 | 164 | 2 | 55 | 55 | 64 |
| 56 | 80 | 20 | 40 | 8 | 25 | 191 | 4 | 82 | 29 | 38 |
| 57 | 20 | 40 | 40 | 8 | 25 | 281 | 0.5 | 26 | 14 | 81 |
| 58 | 20 | 20 | 40 | 4 | 25 | 368 | 1 | 47 | 15 | 85 |
| 59 | 50 | 30 | 30 | 6 | 25 | 228 | 1.7 | 37 | 49 | 62 |
| 60 | 50 | 30 | 30 | 6 | 25 | 228 | 1.7 | 37 | 49 | 62 |
| 61 | 20 | 20 | 40 | 8 | 25 | 368 | 1 | 58 | 30 | 68 |
| 62 | 20 | 20 | 20 | 8 | 25 | 368 | 1 | 34 | 32 | 74 |
| 63 | 50 | 30 | 10 | 6 | 25 | 228 | 1.7 | 90 | 48 | 67 |
| 64 | 110 | 30 | 30 | 6 | 25 | 145 | 3.7 | 61 | 52 | 63 |
| 65 | 50 | 50 | 30 | 6 | 25 | 191 | 1 | 30 | 54 | 67 |
| 66 | 50 | 10 | 30 | 6 | 25 | 281 | 5 | 100 | 3 | 27 |
| 67 | 0 | 30 | 30 | 6 | 25 | 531 | 0 | 0 | 0 | 0 |
| 68 | 50 | 30 | 30 | 6 | 25 | 228 | 1.7 | 31 | 54 | 67 |
| 69 | 50 | 30 | 30 | 6 | 25 | 228 | 1.7 | 31 | 54 | 67 |
| 70 | 50 | 30 | 30 | 10 | 25 | 228 | 1.7 | 58 | 44 | 52 |
| 71 | 50 | 30 | 30 | 2 | 25 | 228 | 1.7 | 68 | 56 | 77 |
| 72 | 50 | 30 | 50 | 6 | 25 | 228 | 1.7 | 63 | 54 | 64 |

Having thus described a few particular embodiments of the invention, various alterations, modifications, and improvements will readily occur to those skilled in the art. Such alterations, modifications, and improvements, as are made obvious by this disclosure, are intended to be part of this description though not expressly stated herein, and are intended to be within the spirit and scope of the invention. Accordingly, the foregoing description is by way of example only, and not limiting. The invention is limited only as defined in the following claims and equivalents thereto.

The subject matter encompassed by the following numbered embodiments also forms part of the present invention, optionally in combination with the subject matter described above and/or defined in the claims that follow.

### NUMBERED EMBODIMENTS

### Numbered Embodiment 1

A method of preparing fluorinated organic compounds comprising contacting hydrogen fluoride with at least one compound of Formula (I):

CX₃CXYCH₃ (I)

where each X is independently Cl, I or Br, and each Y is independently H or F, said contacting step being carried out under conditions effective to produce a compound of Formula (II)

CF₃CZ=CH₂ (II)

where Z is Cl, I, Br, or F.

### Numbered Embodiment 2

The method of Numbered Embodiment 1 wherein said compound of Formula (I) is formed by a chlorinating step comprising contacting a compound of Formula (III)

CHₙX₃₋ₙCXYCH₃ (III)

where n is 0, 1 or 2, with a chlorinating agent.

### Numbered Embodiment 3

The method of Numbered Embodiment 2 wherein said chlorinating agent comprises chlorine gas.

### Numbered Embodiment 4

The method of Numbered Embodiment 2 wherein said chlorinating step is conducted in the presence of light.

### Numbered Embodiment 5

The method of Numbered Embodiment 4 wherein said light has a wavelength of from about 250 to 400 angstoms.

### Numbered Embodiment 6

The method of Numbered Embodiment 2 wherein said chlorinating step is carried out at a temperature of from about 20°C to about 50°C.

### Numbered Embodiment 7

The method of Numbered Embodiment 2 wherein said chlorinating agent comprises chlorine gas and a diluent.

### Numbered Embodiment 8

The method of Numbered Embodiment 2 wherein said compound of Formula III comprises a compound of Formula (IIIA)

CH₃CXYCH₃ (IIIA).

### Numbered Embodiment 9

The method of Numbered Embodiment 2 wherein said compound of Formula III comprises a compound of Formula (IIIB)

CH₂ClCHFCH₃ (IIIB).

### Numbered Embodiment 10

The method of Numbered Embodiment 2 wherein said compound of Formula III comprises a compound of Formula (IIIC)

CH₂ClCHClCH₃ (IIIC).

### Numbered Embodiment 11

The method of Numbered Embodiment 2 wherein said compound of Formula III is formed by contacting a compound of Formula (IV)

CHₙX₃₋ₙCHC=CH₂ (IV)3

With a compound of formula XmY₂-m where m is 0, 1 or 2 and provided that the compound is not H₂.

### Numbered Embodiment 12

The method of Numbered Embodiment 11 wherein said compound of formula XₘY₂₋ₘ comprises HF.

### Numbered Embodiment 13

The method of Numbered Embodiment 11 wherein said compound of formula XₘY₂₋ₘ comprises Cl₂.

### Numbered Embodiment 14

The method of Numbered Embodiment 11 wherein said compound of formula XₘY₂₋ₘ comprises ClF.

### Numbered Embodiment 15

The method of Numbered Embodiment 11 wherein said compound of Formula IV comprises propylene.

### Numbered Embodiment 16

The method of Numbered Embodiment 15 wherein said compound of formula XₘY₂₋ₘ is selected from the group consisting of ClF, HF, Cl₂ and combinations of two or more of these.

### Numbered Embodiment 17

The method of Numbered Embodiment 16 wherein said compound of formula XₘY₂₋ₘ comprises HF.

### Numbered Embodiment 18

The method of Numbered Embodiment 17 wherein said step of contacting a compound of Formula IV comprises conducting at least a portion of said contacting step at a temperature of from about 70°C to about 100°C.

### Numbered Embodiment 19

The method of Numbered Embodiment 17 wherein step of contacting a compound of Formula IV comprises conducting at least a portion of said contacting step at a pressure of from about 5 to about 150 psia.

### Numbered Embodiment 20

The method of Numbered Embodiment 17 wherein said step of contacting a compound of Formula IV comprises conducting at least a portion of said contacting step in the gas phase.

### Numbered Embodiment 21

The method of Numbered Embodiment 17 wherein said step of contacting a compound of Formula IV comprises conducting at least a portion of said contacting step in the gas phase and in the presence of a catalyst.

### Numbered Embodiment 22

The method of Numbered Embodiment 17 wherein said catalyst comprises an iron-based catalyst.

### Numbered Embodiment 23

The method of Numbered Embodiment 17 wherein said catalyst comprises iron chloride.

### Numbered Embodiment 24

The method of Numbered Embodiment 17 wherein said catalyst comprises from about 4 to about 6 percent by weight of iron (III) chloride on a carbon support.

### Numbered Embodiment 25

The method of Numbered Embodiment 16 wherein said compound of formula XₘY₂₋ₘ comprises CIF.

### Numbered Embodiment 26

The method of Numbered Embodiment 25 wherein said step of contacting a compound of Formula IV comprises conducting at least a portion of said contacting step at a temperature of from about -95°C to about -30°C.

### Numbered Embodiment 27

The method of Numbered Embodiment 25 wherein said step of contacting a compound of Formula IV comprises conducting at least a portion of said contacting step at a pressure of from about 5 to about 150 psia.

### Numbered Embodiment 28

The method of Numbered Embodiment 25 wherein said step of contacting a compound of Formula IV comprises conducting at least a portion of said contacting step in the liquid phase.

### Numbered Embodiment 29

The method of Numbered Embodiment 1 wherein said contacting step comprises conducting at least a portion of said contacting step at a temperature of from about 100°C to about 600°C.

### Numbered Embodiment 30

The method of Numbered Embodiment 1 wherein said contacting step comprises conducting at least a portion of said contacting step at a pressure of from about 10 to about 120 psia.

### Numbered Embodiment 31

The method of Numbered Embodiment 1 wherein said contacting step comprises conducting at least a portion of said contact step in the gas phase.

### Numbered Embodiment 32

The method of Numbered Embodiment 1 wherein said contacting step comprises conducting at least a portion of said contact step in the gas phase and in the presence of a catalyst.

### Numbered Embodiment 33

The method of Numbered Embodiment 1 wherein said catalyst comprises nickel.

### Numbered Embodiment 34

The method of Numbered Embodiment 17 wherein said catalyst comprises iron chloride.

### Numbered Embodiment 35

The method of Numbered Embodiment 17 wherein said catalyst comprises from about 4 to about 6 percent by weight of iron (III) chloride on a carbon support.

## Claims

1. A method of preparing fluorinated organic compounds comprising contacting hydrogen fluoride with at least one compound of Formula (I):
CX₃CXYCH₃ (I)
where each X is independently Cl, I or Br, and each Y is independently H or F, said contacting step being carried out under conditions effective to produce a compound of Formula (II)
CF₃CZ=CH₂ (II)
where Z is Cl, I, Br, or F.

2. A method according to claim 1, wherein the at least one compound of Formula (II) comprises 2,3,3,3-tetrafluoro-1-propene (HFO-1234yf).

3. A method according to claim 1, wherein the at least one compound of Formula (II) comprises 2-chloro-3,3,3-trifluoro-1-propene (HFO-1233xf).

4. A method according to any preceding claim, which comprises a liquid phase non-catalytic reaction.

5. A method according to any of claims 1 to 3, which comprises a gas phase catalyzed reaction.

6. A method according to claim 5, wherein the reaction is catalyzed by a catalyst selected from transition metal-based catalysts, such as FeCl₃, chromium oxyfluoride, Ni, NiCl₂ and CrF₃; carbon based catalysts; antimony-based catalysts, such as SbCl_{5;} aluminium-based catalysts, such as AlF₃ and Al₂O₃; palladium-based catalysts; platinum-based catalysts; rhodium-based catalysts; ruthenium-based catalysts; and combinations thereof.

7. A method according to claim 6, wherein the catalyst has been pre-treated by fluorination, prior to use.

8. A method according to any preceding claim, wherein X is Cl.

9. A method according to any preceding claim, wherein compound (I) comprises 2-fluoro-2,3,3,3-tetrachloropropane (HCFC-241bb).

10. A method according to claim 1, wherein said compound of Formula (I) is formed by a chlorinating step comprising contacting a compound of Formula (III)
CHₙX₃₋ₙCXYCH₃ (III)
where n is 0, 1 or 2, with a chlorinating agent, preferably wherein said chlorinating agent comprises chlorine gas.

11. A method according to claim 10, wherein said chlorinating step is conducted in the presence of light, preferably wherein said light has a wavelength of from about 250 to 400 Angstoms.

12. A method according to claim 10, wherein said chlorinating step is carried out at a temperature of from about 20°C to about 50°C.

13. A method according to any of claims 10 to 12, wherein said compound of Formula III is selected from CH₃CXYCH₃ where X and Y are as defined in claim 1, such as CH₃CHFCH₃; CH₂ClCHFCH₃; and CH₂ClCHClCH₃.

14. A method according to any of claims 10 to 13, wherein said compound of Formula III is formed by contacting a compound of Formula (IV)
CHₙX₃₋ₙCHC=CH₂ (IV)
with a compound of formula XₘY₂₋ₘ wherein m is 0, 1 or 2 and provided that the compound is not H₂, preferably wherein said compound of formula XₘY₂₋ₘ is selected from HF, Cl₂, ClF, and combinations thereof.

15. A method according to claim 14, wherein said compound of Formula IV comprises propylene.
